# EUROPEAN PATENT APPLICATION

(11) **EP 2 186 532 A1**
(43) Date of publication of application: **19.05.2010**
(21) Application number: 08019733.8
(22) Date of filing: 12.11.2008
(51) Int. Cl.: A61M 1/06

(54) **Breast pump**

(71) Applicant: Ardo medical AG, 6314 Unterägeri (CH)
(72) Inventor: Speck, Richard, 6038 Gisikon (CH)
(74) Representative: Fischer, Britta Ruth

(57) **Abstract**

The invention relates to a breast pump (10; 30) comprising a body (15) with an inlet (16) thereto to receive milk expressed from a woman's breast, means (17) for releasably attaching the breast pump (10; 30) to a container (7) for collecting expressed milk, a membrane (5.1, 5.2) or a flexible bellow (12) mounted on the body (15), the membrane (5.1, 5.2) or the flexible bellow (12) in use being cyclicly moved by actuating means (4) connected to it, wherein the actuating means (4) are connectable to a cover (3; 11) that is mounted on the body (15) such that it is rotatable around the longitudinal axis (19) of the breast pump (10; 30) and that the body (15) and the cover (3; 11) are designed such that the cover (3; 11) can be fastened firmly on the body (15) in several pre-defined locations around its periphery.

## Description

The invention relates to a breast pump according to the preamble of claim 1 which can either be manually operated or electrically driven.

From European patent application EP-A2-0733 376 A2 there is known a single-handed breast pump with a body with a milk inlet, wherein the pump is releasably attached to a milk collecting container. In the body is mounted a deformable diaphragm which in use is cyclicly moved by actuating means. Valve means in the body are operable such that a negative pressure is cyclicly generated and released in the nipple region to stimulate the nipple area to cause lactation. For manual operation a moulded plastics lever is provided that comprises a handle connected to a nose portion having a forked end. By raising the noise portion a connector is lifted and the diaphragm becomes distorted. The handle is neither fastened at the cap nor can it be rotated around the longitudinal axis of the breast pump. For holding the diaphragm a circular moulded rigid plastics diaphragm holder with a generally frusto conical cross sectional shape is provided. The external contours of the holder are shaped so that they fit exactly inside the diaphragm and hold it against the body to make fluid tight seal therewith.

Further known modular breast pumps are so called two-handed breast pumps, where both hands have to be used for application, one hand holding the milk-receiving container and the other hand operating the manual suction device, i.e. the lever. The flexibility of the woman applying such a two handed breast pump is limited with respect to the use of her hands. Other known modular breast pumps utilize for force transmission and hence suction generation a parallelogram of force instead of a lever with two-point bearing. Utilizing a parallelogram of force makes the application of the breast pump, in particular its tightness, user dependent.

Often a snap-on fixture (or simple overlapping) is applied and snapped onto the body of a breast pump, thereby clamping the outer rim of a pot-shaped diaphragm. Before snapping on the snap-on fixture onto the opening of the body a vacuum has to be generated to ensure a restraining force of the pot-shaped diaphragm which is cumbersome. The pot-shaped diaphragm has to be mounted such that its outer rim is drawn over the upper part of the body forming its opening and it has to be avoided that the rim of the diaphragm slip-slides away from this upper part of the body. The snap-on fixture is usually combined with a compressible piston to generate suction on the diaphragm. While the snap-on fixture can be rotated around the longitudinal axis of the breast pump, it can only be rotated continuously and not be fastened in one particular position which makes positioning inaccurate.

Using a screw coupling for holding the pot-shaped diaphragm makes the tightness of the breast pump user dependent.

It is an object of the invention to provide a single-handed breast pump by which the above-mentioned drawbacks can be avoided, the breast pump being either manually operable or electrically drivable. It is a further object of the invention to provide a breast pump that can be equally well applied by a left-handed person or a right-handed person.

In order to implement these and still further objects of the invention, which will become more readily apparent as the description proceeds, a breast pump is provided that comprises a body with an inlet thereto to receive milk expressed from a woman's beast, means for releasably attaching the breast pump to a container (e.g. a bottle) for collecting expressed milk, a membrane (also called diaphragm) or a flexible bellow mounted on the body, the membrane or the flexible bellow in use being cyclicly moved by actuating means connected to it, wherein the actuating means are connectable to a cover that is mounted on the body such that it is rotatable around the longitudinal axis of the breast pump and wherein the body and the cover are designed such that the cover can be fastened firmly on the body in several pre-defined locations around its periphery. Preferably the cover is designed such that it holds the membrane or the flexible bellow, respectively, in position on the body. For mounting the membrane or the flexible bellow the generation of a vacuum is advantageously not required.

The breast pump according to the invention can be used as manually operated one-handed breast pump with a lever as actuating means or it can have a modular design for combination with a motorized drive unit to form an electric breast/milk pump. In case of the breast pump being manually operated, the lever (and hence its pivotal point) is positioned at the cover and not at the body (e.g. its breast shield) of the breast pump.

According to a preferred embodiment of the invention the cover and the body are mechanically connected by means of a bayonet connection with several pins on the male side and several matching slots on the female side of the bayonet connector (confer http://en.wikipedia.org/wiki/Bayonet_mount), wherein the female side and the male side are interchangeable. Preferentially, there are three pins (or set of pins) on the male side and three matching slots (or set of slots) on the female side, with preferably one pin and one slot (or one set of pins and one set of slots) being located opposite the inlet of the breast pump and the other two pins and slots (or two sets of pins and slots) being located on opposing side in-between the inlet and the one pin and slot (one set of pins and slots) positioned opposite the inlet, thereby defining three positions for the actuating means, in particular the lever, namely opposite the inlet or on either the right hand side or the left hand side of the inlet. This has the advantage ergonomic considerations can be taken into account when positioning the lever/Cover (actuating means) of the breast pump and that the breast pump can be used equally well and basically effortlessly by a left hander and a right hander for pumping milk. Of course, the pins and there corresponding slots can be arranged equally spaced.

Using a bayonet connection has the further advantage that the amount of fixation of the cover to the body and hence the tightness of this connection (and hence of the breast pump body) are basically user independent. The bayonet connection between the cover and the body provides a secure and well defined mechanical connection, which ensures optimal sealing and holding of the membrane (or flexible bellow) in-between the cover and the body. Sealing can be achieved more determinately and is easier to handle as with in particular snap-on fixtures.

Alternatively, the cover and the body can also be connected by means of a screw coupling with several spaced apart thread part sections (which may also be considered as segments) on the male side and several matching helix part sections (also called segments) on the female side of the screw coupling. Female side and male side are interchangeable. Preferably there are three thread part sections on the male side and three matching helix part sections on the female side, which may be located as described above for the bayonet connection.

Further advantageous features and applications of the invention can be found in the dependent claims as well as in the following description of the drawings illustrating the invention. In the drawings like reference signs designate the same or similar parts throughout the several figures of which:
Fig. 1 shows a manually operated breast pump according to the invention in front view (Figure 1a)) and in sectional side view (Figure 1b)) along the line B-B of Figure 1a),
Fig. 2 shows an electrically drivable breast pump according to the invention in front view (Figure 2a)) and in sectional side view (Figure 2b)) along the line A-A of Figure 2a),
Fig. 3 shows a membrane of the manually operated breast pump depicted in Figure 1 in front view (Figure 3a)), in sectional side view (Figure 3b)) along the line A-A of Figure 3a, and in perspective view from above (Figure 3c)), and
Fig. 4 shows the manually operated breast pump depicted in Figure 1 with the bayonet connection between the cover and the body, wherein Figure 4a) shows in top view the cover being rotated, Figure 4b) shows in top view the cover being firmly fastened, and Figure 4c) shows Figure 4b) in perspective view from above.

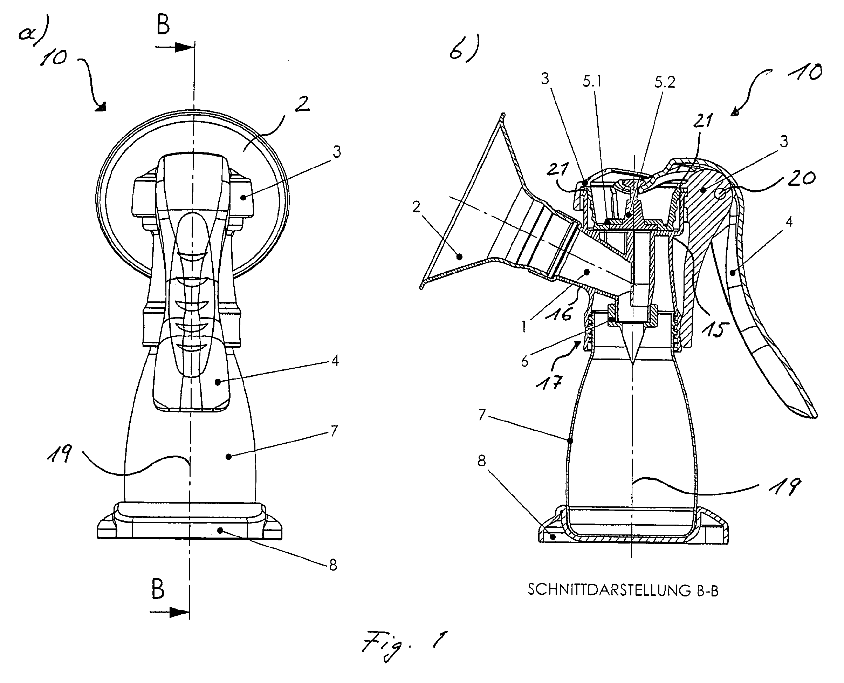

Figure 1 shows a manually operated breast pump 10 according to the invention in front view (Figure 1a) and in sectional view along the line B-B of Figure 1a) (Figure 1b)). The depicted breast pump 10 is a one-handed lever breast pump. The breast pump 10 comprises of body 15 with an inlet 16. The inlet 16 forms a breast shield base 1 to which a breast shield top 2 is releasably attached. Breast shield tops of various types and sizes can be attached - via an appropriate conical plug-in adapter or directly if possible - to the breast shield base 1 to allow use of the breast pump by differently sized women. The breast shield top 2 can be attached by plugging it into the breast shield base 1. The breast shield base 1 forms together with the breast shield top 2 the entire breast shield. The breast shield top 2 preferentially has the shape of an inlet trumpet or funnel, respectively.

The body 15 is connected via a screw joint 17 to a container 7 for collecting expressed milk. The container 7 preferably has already the form of a bottle which can be used for feeding an infant. A container stand 8, in particular in form of a bottle stand, is provided in which the container 7 can be placed or to which it is attached so that the container 7 (and also the breast pump 10) can be prevented from toppling over.

Between the body 15 and the container 7 there is provided a valve 6, in particular a lip valve, functioning as a check valve allowing fluid only to flow through it from the body 15 (or its inlet 16, respectively) to the container 7.

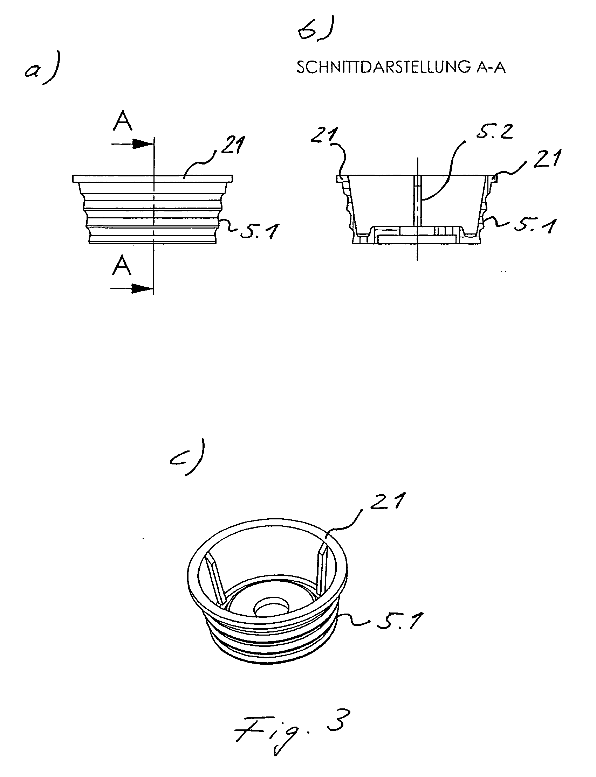

In the body 15 is mounted as membrane a pot-shaped membrane 5.1 with a membrane con rod 5.2. The pot-shaped membrane 5.1 can for example be made of silicon. The membrane con rod 5.2 is in particular integrally formed with the pot-shaped membrane 5.1. Figure 3 shows an example of such a membrane comprising a pot-shaped membrane 5.1 and a membrane con rod 5.2, wherein the pot-shaped membrane 5.1 has an upper rim 21. The pot-shaped membrane 5.1 is arranged such that its bottom part runs transverse to the longitudinal axis 19 of the breast pump 10 and is positioned on an abutment of the body 15. The membrane con rod 5.2 is arranged in the middle of the pot-shaped membrane 5.1 and extends upwardly from it toward the actuating means 4, which for the manually operated breast pump 10 are given by a two-point lever 4 that can be pivoted around the pivotal point 20 relative to the body 15. The lever 4 is connected to a cover 3 of the breast pump 10.

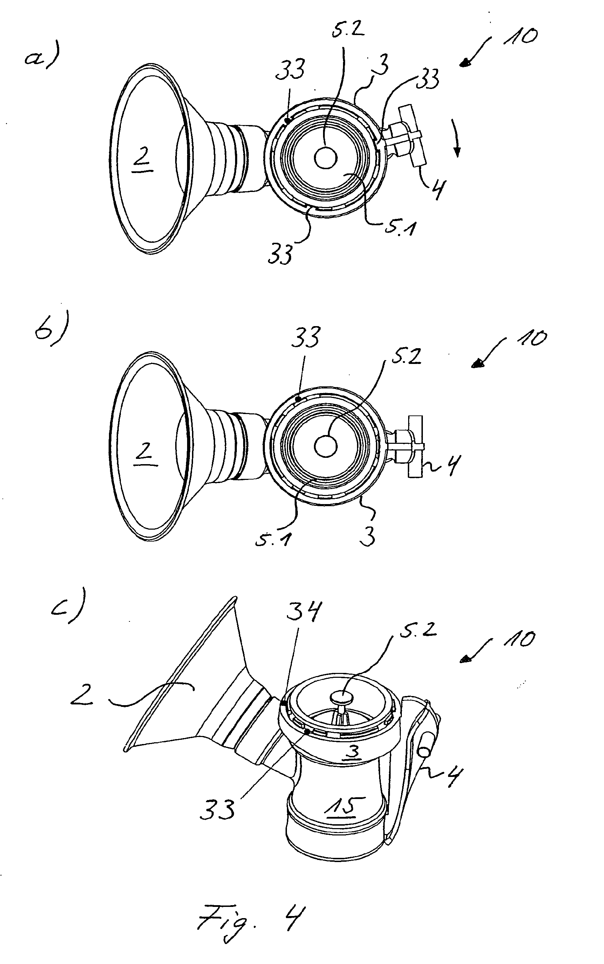

The lever 4 is clicked onto/attached to the cover 3 and can be removed e.g. for cleaning. The cover is formed as casing cover 3 and it is rotatably mounted on the body 15. By rotating the casing cover 3 around the longitudinal axis 19 of the breast pump 10, also the lever 4 is rotated. The upper rim 21 of the pot-shaped membrane 5.1 is positioned between the body 15 (its upper part) and the casing cover 3 such that the rim 21 of the pot-shaped membrane 5.1 is clamped between the casing cover and the body 15 when they are connected. The casing cover 3 and the body 15 are preferably connected by bayonet connection which is further described with respect to Figure 4.

In use the user first inserts her breast and nipple into the open end of the breast shield top 2 and holds with one hand the body 15 and the container 7 against her breast. The fingers of the same hand then move the lever 4 about the pivotal point 20 against the resilience of the pot-shaped membrane 5.1, thereby lifting the membrane con rod 5.2 upwardly as can be derived from Figure 1b). Raising the membrane con rod 5.2 distorts/moves the pot-shaped membrane 5.1 connected thereto upwardly from the abutment of the body 15, thereby creating a vacuum or negative pressure in the breast shield 1, 2 between the breast and the lip valve 6, which is also lifted upwardly and forms a seal to maintain the negative pressure in the body 15. This negative pressure then causes the breast to lactate such that milk is expressed therefrom. Releasing the lever 4, the resilience of the pot-shaped membrane 5.1 causes the membrane 5.1, 5.2 to return to its initial position. The negative pressure is released and the lip valve 6 opens allowing the expressed milk to flow under gravity into the container 7. The operation of the lever 4 is repeated cyclicly to create alternating negative pressure in the nipple region in order to assist lactation.

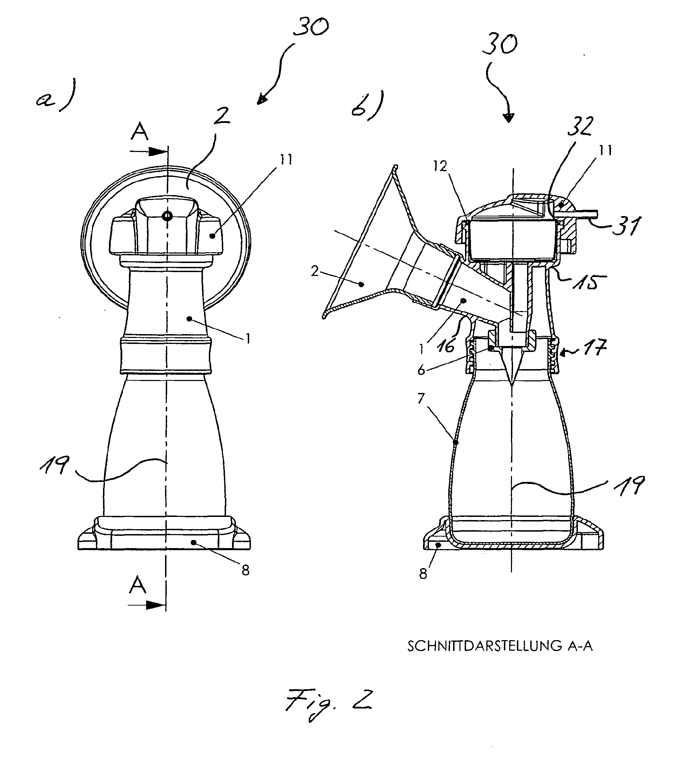

Figure 2 shows an electrically drivable breast pump 30 according to the invention in front view (Figure 2a)) and in sectional side view (Figure 2b)) along the line A-A of Figure 2a) (Figure 2b)). For parts or components which also form part of the manually operated breast pump 10 shown in Figure 1 it is referred to the above description of Figure 1.

The breast pump 30 shown in Figure 2 can be operated as an electric breast pump. For this a flexible bellow 12 (also called vacuum pot) is used as membrane, which also serves as check valve. The bottom of the flexible bellow 12 runs transverse to the longitudinal axis 19 of the breast pump 30 and rests on an abutment of the body 15. The cover is formed as adapter tube cover 11 with a tubing 31, i.e. as a cover that serves via a tubing 31 as adapter for separate actuating means. As actuating means (not shown) serves a motorized drive unit that comprises an electric motor which in use cyclicly supplies suction through the tubing 31 to the body 15. The suction moves the flexible below 12 upwardly away from the abutment of the body 15, seals the lip valve 6 and generates a vacuum or negative pressure between the breast inside the breast shield 1, 2 and the lip valve 6. A lever is obsolete.

The adapter tube cover 11 and the body 15 are connected through bayonet connection, thereby clamping the upper rim 32 of the flexible bellow 12 (in analogy to the bayonet connection between the casing cover 3 and the body 15 of the manually operated breast pump 10).

Fig. 4 shows the manually operated breast pump 10 depicted in Figure 1 with the bayonet connection between the cover 3 and the body 15, wherein Figure 4a) shows in top view the cover during rotation, Figure 4b) shows in top view the cover being firmly fastened, and Figure 4c) shows Figure 4b) in perspective view from above. The manually operated breast pump 10 has only been chosen for exemplarily reasons. The teachings of Figure 4 apply equally well to the electrically drivable breast pump 30 depicted in Figure 2. For ease of presentation the upper side of the cover 3 (seen from the container stand 8 as lowest point) has been removed (which is indicated by reference sign 34), so that the membrane 5.1, 5.2 and the bayonet connection can be seen.

For the bayonet connection the cover 3 comprises for example three pins (not shown) and the body 15 comprises tree corresponding slots (not shown). For coupling of the cover 3 with the body 15 the user has to align the pins with the slots and rotate either the cover 3, or the body 15 or both of them in opposite directions in order to guide the pins into perpendicular slots which prevent the pins from being removed. The positions of the pins and slots define the possible positions of the lever 4 (or the tubing 31, respectively). In the example of Figures 4b) and 4c) the lever is firmly fastened opposite the breast shield top by the bayonet connection.

For clamping the rim 21 of the pot-shaped membrane 5.1 (or the rim 32 of the flexible bellow 12, respectively) while closing the bayonet connection the cover 3 (or the adapter tube cover 11, respectively) preferably has clamping cams 33 extending inwardly to further clamp the rim 21 of the pot-shaped membrane 5.1 between the cover 3 and the body 15. While closing the bayonet connection the clamping cams 33 are themselves squeezed against the inside of the cover 3 (as shown in Figure 4b)). Preferably, there are provided three sets of clamping cams 33 equally distributed around the inner periphery of the cover 3, each set of clamping cams 33 having for example three clamping cams 33.

## Claims

1. A breast pump comprising a body (15) with an inlet (16) thereto to receive milk expressed from a woman's breast, means (17) for releasably attaching the breast pump (10; 30) to a container (7) for collecting expressed milk, a membrane (5.1, 5.2) or a flexible bellow (12) mounted on the body (15), the membrane (5.1, 5.2) or the flexible bellow (12) in use being cyclicly moved by actuating means (4) connected to it, **characterised in that** the actuating means (4) are connectable to a cover (3; 11) that is mounted on the body (15) such that it is rotatable around the longitudinal axis (19) of the breast pump (10; 30) and that the body (15) and the cover (3; 11) are designed such that the cover (3; 11) can be fastened firmly on the body (15) in several pre-defined locations around its periphery.

2. The breast pump according to claim 1, wherein the cover (15) is designed such that it holds the membrane (5.1, 5.2) or the flexible bellow (12) in position on the body (15).

3. The breast pump according to claim 1 or 2, wherein the cover (3; 11) and the body (15) are connectable by means of a bayonet connection with several pins on the male side and several matching slots on the female side, in particular with three pins on the male side and three matching pins on the female side.

4. The breast pump according to claim 1 or 2, wherein the cover (3; 11) and the body (15) are connectable by means of a screw coupling with several spaced thread part sections on the male side and several matching helix part sections on the female side of the coupling, in particular with three thread part sections on the male side and three matching helix part sections on the female side.

5. The breast pump according to one of the preceding claims, wherein the membrane (5.1, 5.2) is a pot-shaped membrane (5.1) with a membrane con rod (5.2) that is in particular integrally formed with the pot-shaped membrane (5.1), wherein the membrane con rod (5.2) extends upwardly from the pot-shaped membrane (5.1) toward the actuating means (4).

6. The breast pump according to one of the preceding claims, wherein the cover is designed as a casing cover (3) for the body (15) and the actuating means for cyclicly moving the membrane (5.1, 5.2) are given by a manually movable lever (4) which is pivotally mounted on the casing cover (3) for movement relative to the body (15).

7. The breast pump according to claim 6, wherein in use the lever (4) is moved against the resilience of the membrane (5.1, 5.2).

8. The breast pump according to one of the claims 1 to 4, wherein the cover is designed as an adapter tube cover (11) and the actuating means for cyclicly moving the flexible bellow (12) are given by a motorized drive unit that comprises an electric motor which in use supplies suction through a tubing (31) to the body (15).

9. The breast pump according to one of the preceding claims, wherein the cover (3; 11) has at its inner periphery clamping cams (33) extending inwardly for clamping the membrane (5.1; 5.2; 11) to the body (15).

10. The breast pump according to one of the preceding claims, wherein a breast shield (1, 2) is attached to and/or at least partly formed by the inlet (16) of the body (15), the breast shield (1, 2) comprising a breast shield top (2) in form of a funnel and a breast shield base (1), and wherein in particular the breast shield top (2) is releasably connected to the breast shield base (1).
